# EUROPEAN PATENT APPLICATION

(11) **EP 2 283 779 A1**
(43) Date of publication of application: **16.02.2011**
(21) Application number: 10251414.8
(22) Date of filing: 09.08.2010
(51) Int. Cl.: A61B 17/068, A61B 19/00

(54) **Surgical stapler with visual positional indicator**

(30) Priority: 11.08.2009 US 232843 P; 02.06.2010 US 792221
(71) Applicant: Tyco Healthcare Group LP, New Haven, CT 06511 (US)
(72) Inventor: Zemlok, Michael A., Prospect, CT 06712 (US); Ross, Adam J., Prospect, CT 06712 (US)
(74) Representative: Soames, Candida Jane

(57) **Abstract**

The present disclosure is directed to a surgical stapler for applying sterilized staples to body tissue. The surgical stapler includes a cartridge housing a plurality of fasteners, an anvil mounted adjacent the cartridge, a drive assembly configured to translate through the cartridge to eject the fasteners therefrom, and at least one visual indicator configured to indicate a position of the drive assembly, wherein the visual indicator includes a material that provides an optical contrast with at least one of the cartridge and the anvil.

## Description

### CROSS REFERENCE TO RELATED APPLICATION

The present application claims the benefit of and priority to U.S. Provisional Application Serial No. 61/232,843 filed on August 11, 2009, the entire contents of which are incorporated herein by reference.

### BACKGROUND

### 1. Technical Field

The present disclosure relates to surgical stapling instruments, and more particularly, to a position indicator. Even more particularly, the present disclosure relates to a surgical stapling device that has a visual reference for the sealing or cutting position enhanced by phosphorescence.

### 2. Background of Related Art

Surgical stapling devices wherein tissue is first grasped or clamped between opposing jaw structure and then joined by means of surgical fasteners are well known in the art. In some instruments, a knife is provided to cut the tissue which has been joined by the fasteners. The fasteners are typically in the form of surgical staples or two part fasteners.

Instruments for this purpose include two elongated members which are respectively used to capture or clamp tissue. Typically, one of the members carries a cartridge which houses a plurality of staples arranged in two lateral rows while the other member comprises an anvil that which defines a surface for forming the staple legs as the fasteners are driven from the cartridge. Generally, the stapling operation is effected by a pusher which travels longitudinally through the cartridge carrying member, with the pusher acting upon the staples to sequentially eject them from the cartridge. A knife may be associated with the pusher between the staple rows to longitudinally cut and/or open the tissue disposed between the rows of staples. Such instruments are disclosed, for example, in commonly assigned U.S. Pat. Nos. 3,079,606, 3,490,675 and 5,014,899.

A later stapler disclosed in U.S. Pat. No. 3,499,591 to Green applies a double row of staples on each side of the incision. This is accomplished by providing a cartridge assembly in which a cam member moves through an elongate guide path between two sets of staggered staple carrying grooves. Staple drive members are located within the grooves and are positioned in such a manner so as to be contacted by the longitudinally moving cam to effect ejection of the staples.

Each of the instruments described above were designed for use in open surgical procedures wherein surgeons have direct manual access to the operative site. However, in endoscopic or laparoscopic procedures, surgery is performed through a small incision or through narrow cannula inserted through small entrance wounds in the skin. In order to address the specific needs of endoscopic and/or laparoscopic surgical procedures, an endoscopic surgical stapling apparatus has been developed and is disclosed in U.S. Pat. No. 5,040,71 to Green et al. This apparatus is well suited for such procedures and includes a fastener applying assembly having an anvil and a staple cartridge provided at the distal end of an endoscopic portion which permits the instrument to be inserted into a cannula and be remotely operated by the surgeon through manipulation of a proximal handle mechanism.

During the performance of a surgical stapling procedure, it is necessary for the surgeon to be well aware of the dimensional limitations of the instrument which they are using. For example, when tissue is to be stapled and cut, the surgeon must be able to accurately position the tissue within the jaws of the apparatus to ensure the tissue will be properly stapled and cut. In the past, markings have been imprinted on the cartridge carrying member of such stapling instruments. See, for example, U.S. Pat. Nos. 4,633,874, Des. 272,851, Des. 278,080 and Des. 284,698. A later stapler disclosed in U.S. Pat. No. 5,560,530 to Bolanos et al. provides a graduated anvil.

### SUMMARY

The present disclosure is directed to a surgical stapling instrument. More particularly, the surgical stapling instrument has visual indicators that provide the operator with information relating to the position of a movable part.

In one embodiment, the surgical stapling instrument has a cartridge, an anvil, a drive assembly, and the visual indicator. The cartridge contains a plurality of fasteners and is mounted adjacent the anvil. The drive assembly moves through the cartridge to eject the fasteners from the cartridge. The visual indicator has a material that provides an optical contrast to the body of either, or both, the cartridge and the anvil.

The visual indicator provides the surgeon with information concerning one or more of the following: clamp position, overall travel distance, proximal limit, and distal limit. This information is conveyed through an optical contrast provided by contrasting colors, variation in reflectivity, luminescent coating, or combinations thereof.

To facilitate visualization of the visual indicator, the openings are formed in the anvil and cartridge. The openings may be a slot. Indicia are placed about the openings. The indicia also provide an optical contrast to the body of either, or both, the anvil and the cartridge.

### DESCRIPTION OF THE DRAWINGS

The accompanying drawings, which are incorporated in and constitute a part of this specification, illustrate embodiments of the disclosure, and together with a general description of the disclosure given above, and the detailed description of the embodiments given below, serve to explain the principles of the disclosure.
FIG. 1 is a perspective view of a surgical stapling instrument in accordance with the principles of the present disclosure;
FIG. 2 is an enlarged perspective view of the fastener applying assembly having a series of openings for viewing the visual indicator through, in accordance with the principles of the present disclosure;
FIG. 3 is an enlarged perspective, cross-sectional view of the fastener applying assembly with the drive assembly in the proximal position in accordance with the embodiment of FIG. 1-2;
FIG. 4 is an enlarged perspective, cross-sectional view of the fastener applying assembly with the drive assembly in the distal position in the proximal position in accordance with the embodiment of FIGS. 1-2;
FIG. 5 is an enlarged perspective view of the fastener applying assembly having a slot for viewing the visual indicator through, in accordance with another embodiment of the present disclosure;
FIG. 6 is an enlarged perspective view of the fastener applying assembly having a series of openings on the cartridge for viewing the visual indicator through in the proximal position in accordance with the embodiment of FIGS. 1-2;
FIG. 7 is an enlarged perspective view of the fastener blade having a visual indicator in the proximal position in accordance with the embodiment of FIGS. 1-2; and
FIG. 8 is an enlarged perspective view of a retainer having a visual indicator in the proximal position in accordance with the embodiment of FIG. 5.

Other features and advantages of the present disclosure will become apparent from the following detailed description, taken in conjunction with the accompanying drawings, which illustrate, by way of example, the principles of the present disclosure.

### DETAILED DESCRIPTION OF THE EMBODIMENTS

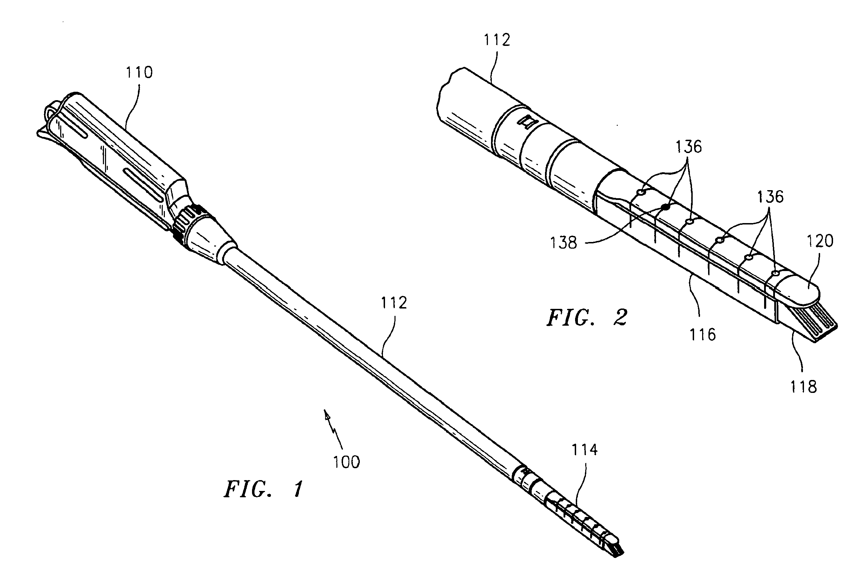

Referring now to the drawings wherein like reference numerals indicate similar structural features of the subject invention, there is illustrated in FIG. 1 a surgical stapling instrument 100 that includes a visual indicator in accordance with the current disclosure.

The surgical stapler 100 is adapted and configured for utilization during endoscopic or laparoscopic procedures wherein surgery is performed through a small incision or entrance wound formed in a patient's body. More particularly, during laparoscopic procedures, surgical stapler 100 can be extended through a trocar or cannula device into a cavity of a patient to sequentially apply a plurality of surgical fasteners to body tissue, and, concomitantly incise the fastened tissue. Surgical stapler 100 includes a handle assembly 110, an elongate body 112 that extends distally from handle assembly 110, and a fastener applying assembly 114 that is operatively associated with a distal end portion of elongate body 112.

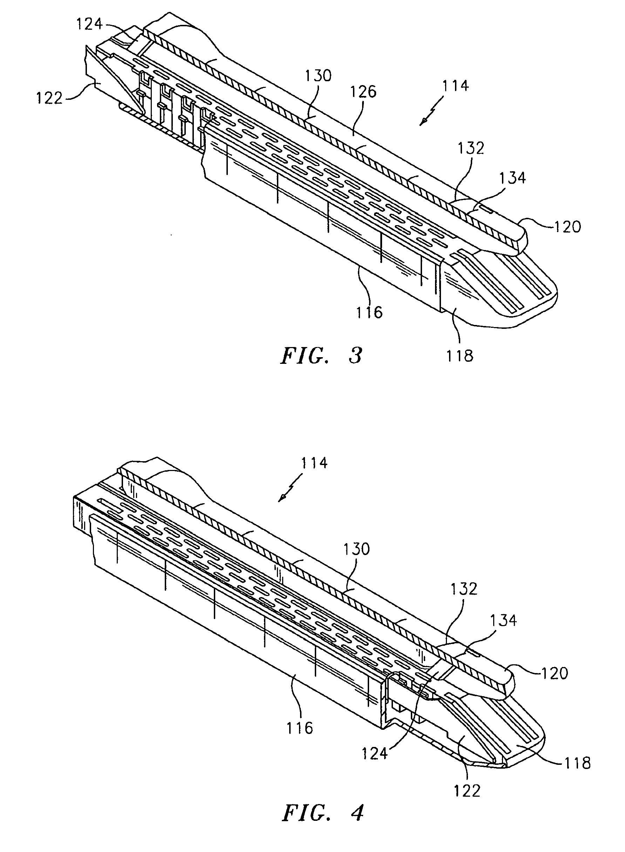

Referring to FIGS. 2-4, fastener applying assembly 114 includes a cartridge housing 116 configured to support a staple carrying cartridge 118, and an anvil 120 mounted adjacent cartridge housing 116. The anvil defines a staple forming surface (not shown). As best seen in FIGS. 3 and 4, a fastener driving mechanism 122 is operatively associated with staple cartridge 118 and is configured to translate through cartridge 118, in a longitudinal direction, to sequentially eject the surgical fasteners therefrom and drive them against the staple forming surface of anvil 120. A knife blade 124 is associated with fastener driving mechanism 122 and is configured to translate through cartridge 118 in conjunction therewith to form an incision in the body tissue situated between the parallel rows of staples applied thereto. In operation, the fastener driving mechanism 122 and the knife blade 124 translate together from the pre-fired proximal position illustrated in FIG. 3 to the post-fired distal position illustrated in FIG. 4.

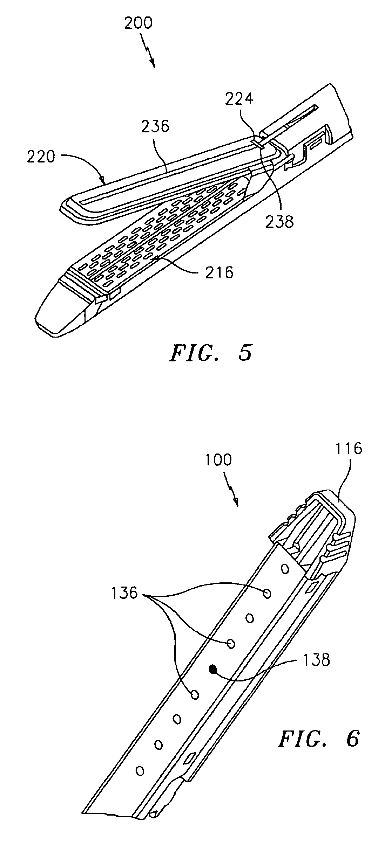

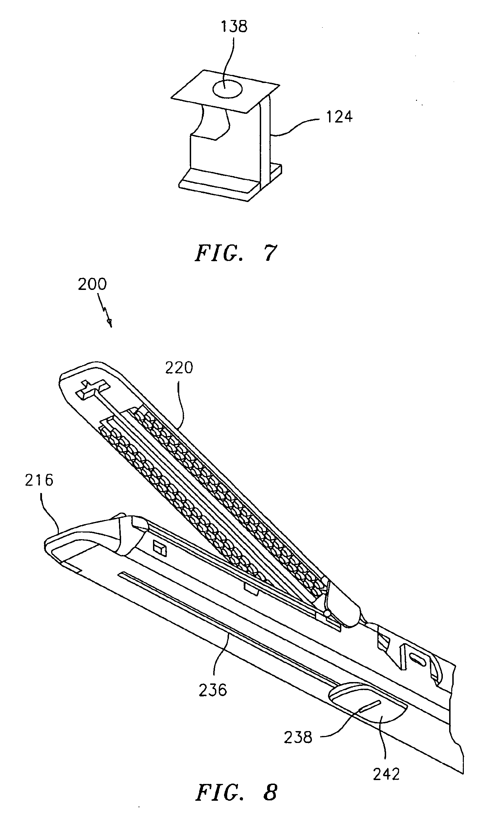

As best shown in FIG. 7, the blade 124 includes a visual indicator 138 located on the outermost surface within the anvil, or top surface. This configuration allows the operator to observe the location of the visual indicator 138 as the drive mechanism 122 and knife blade 124 travel distally along the fastener applying assembly 114 as observed from the top of the surgical stapler 100. As best seen in FIG. 6, an additional visual indicator 138 may be placed on the outermost surface within the cartridge housing 116, or bottom surface. This configuration allows the operator to observe the location of the visual indicator 138 as the drive mechanism 122 and knife blade 124 travel distally along the fastener applying assembly 114 as observed from the bottom of the surgical stapler 100. The visual indicator 138 is visible because it optically contrasts with the surrounding structure.

The optical contrast can be provided by contrasting colors, variations in reflectivity, or a luminescent coating and can be applied by imprinting the surface utilizing inking, painting, staining, etching, or molding a luminescent material in place. Reduced visibility and lighting from minimally invasive laparoscopic or endoscopic procedures provide the operator with difficulties while observing the fastener applying assembly 114. The optical contract can provide light, thereby allowing the operator to see, by using a luminescent coating on the knife blade 124, driving mechanism 122, anvil 120, cartridge housing 116, or any combination thereof. The luminescent coating causes light to be produced from chemical reactions, electrical energy, subatomic motions, or stress on a crystal. Further, any material or treatment of material that produces an optical contrast may be used.

As best seen in FIG. 2, the outer surface 126 of anvil 120 is provided with a series of graduations or markings 130 placed about each of the openings, which correspond to particular linear dimensions of the anvil 120. More particularly, as illustrated in FIGS. 3 and 4, graduations 130 serve to delineate the range through which the fastener driving mechanism 122 travels during a stapling operation. The graduations provide a user with the capability to determine, from a remote point of observation (utilizing a laparoscope), the specific operating range of the staple driving mechanism 122. The visual indicator 138 in conjunction with the markings 130 provide the operator with information relating to drive position, clamp position, overall travel distance, proximal limit, and distal limit, by allowing the operator to visually determine the position of the visual indicator as it moves in reference to fixed locations.

The graduations are defined by linear markings coupled with numerical indicia. In one embodiment, the graduations are spaced in 10mm intervals and the numerical indicia decrease in magnitude in a distal direction. An additional linear marking 132 is provided adjacent to the distal-most graduation 134 to indicate the distal-most boundary of the range through which the knife blade 124 travels during a stapling operations. The linear markings and the indicia are provided with the same optical contrast as the visual indicator.

Referring to FIG. 2, a series of position indicators, or openings 136, on the outside of anvil 120 is shown. The position of the visual indicator 138 in relation to anvil 120 is observable through these openings during the stapling and cutting operation. Referring to FIG. 6, a series of position indicators or openings 136, on the outside of the cartridge 116 is shown. The position of a visual indicator 138 in relation to cartridge 116 is observable through these openings during the stapling and cutting operation.

Referring to FIGS. 5 and 8, another embodiment is shown. The surgical stapler 200 has an opening 236 in the form of a slot along the anvil and the cartridge. In this embodiment, the visual indicator 238 is located on the knife 224 and retainer 242, and is continually observable as the knife 224 travels distally along the fastener applying assembly.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure. Those skilled in the art will envision other modifications within the scope and spirit of the claims appended hereto.

## Claims

1. A surgical tool apparatus for applying sterilized staples to body tissue comprising:
a cartridge housing a plurality of fasteners;
an anvil mounted adjacent the cartridge;
a drive assembly configured to translate through the cartridge to eject the fasteners therefrom; and
at least one visual indicator configured to indicate a position of the drive assembly,
wherein the visual indicator includes a material that provides an optical contrast with at least one of the cartridge and the anvil.

2. The surgical tool of claim 1, wherein the at least one visual indicator provides the surgeon with information selected from a group consisting of clamp position, overall travel distance, proximal limit, and distal limit.

3. The surgical tool of claim 1, wherein the optical contrast is selected from a group consisting of contrasting colors, variations in reflectivity, and luminescent coating.

4. The surgical tool of claim 1, further comprising at least one opening formed on the anvil, wherein the opening is adapted to facilitate visualization of the visual indicator.

5. The surgical tool of claim 4, wherein the at least one opening is a slot.

6. The surgical tool of claim 4, further comprising indicia placed about the at least one opening.

7. The surgical tool of claim 6, wherein the indicia including a material that provides an optical contrast.

8. The surgical tool of claim 7, wherein the optical contrast is selected from a group consisting of contrasting colors, variations in reflectivity, and luminescence coating.
